# EUROPEAN PATENT APPLICATION

(11) **EP 4 764 450 A1**
(43) Date of publication of application: **24.06.2026**
(21) Application number: 25223295.4
(22) Date of filing: 15.12.2025
(51) Int. Cl.: G01N 1/28

(54) **SAMPLE GRINDING KIT, SAMPLE GRINDING DEVICE AND SAMPLE GRINDING METHOD**

(30) Priority: 23.12.2024 TW 113150120
(71) Applicant: Taiwan Advanced Nanotech Inc., Taoyuan City 333 (TW)
(72) Inventor: Chien, Chien-Hsing, 333 Taoyuan City (TW); Mok, Kan-Sang, 333 Taoyuan City (TW)
(74) Representative: Charrier Rapp & Liebau

(57) **Abstract**

The present disclosure provides a sample grinding set, a sample grinding device (100) and a sample grinding method (400) for biological samples, which are particularly suitable for grinding biological samples in pre-processing of nucleic acid extraction. The sample grinding kit (1) combines a grinding piece with a spiral part and a sample tube (10). Through the rotation of the grinding piece, the spiral part drives the sample and grinding auxiliary material (200)s to achieve the effect of grinding the sample.

## Description

### BACKGROUND OF THE INVENTION

### FIELD OF THE INVENTION

The present disclosure relates to sample grinding kits, grinding devices and grinding methods, and more particularly to a sample grinding kit, grinding device and grinding method which are particularly suitable for grinding biological samples in pre-processing of nucleic acid extraction. In particular, the present disclosure relates to grinding biological samples in pre-processing of nucleic acid extraction with a biological sample grinding kit to achieve high-performance, precise and stable biological sample grinding and thereby meet the need for subsequent molecular biology application.

### DESCRIPTION OF THE PRIOR ART

Biological sample grinding is a step critical to the pre-processing process of nucleic acid extraction, because it is effective in breaking open cell walls and membranes to allow nucleic acids to be released from cells. However, existing biological sample grinding techniques are confronted with some common issues that may compromise the efficiency and quality of nucleic acid extraction. The issues are summarized as follows:
1. Sample loss: in the course of grinding, samples may adhere to a grinding tool or splash and thus get lost, reducing nucleic acid yields.
2. Cross-contamination: when multiple samples undergo grinding in the same equipment but the equipment has not yet been thoroughly cleaned, cross-contamination among the samples is likely to occur, devastating downstream application (for example, PCR or gene sequencing) of high-purity and contamination-free nucleic acids.
3. Uneven grinding: manual or mechanical grinding is likely to end up with the uneven or incomplete breaking open of the cell walls and membranes of samples; as a result, nucleic acid extraction is incomplete, compromising nucleic acid extraction efficiency.
4. Temperature control: the heat generated from the grinding process may compromise nucleic acid stability and cause nucleic acid degradation, reducing the quality and quantity of extraction.
5. Equipment abrasion: long-duration or high-intensity grinding causes grinding equipment abrasion; as a result, not only is equipment efficiency compromised, but intrusion of contaminants derived from metals or other abrasion materials also occurs.
6. Sample type limitation: some grinding techniques are inapplicable to specific types of samples (for example, plant tissue with high cellulose content or hard samples), and thus the cell walls and membranes of the samples cannot be effectively broken open.
7. Intricate operation: manual grinding not only entails relatively more operation steps but also has relatively stricter technician operation requirements; as a result, manual grinding is likely to cause inconsistent operation, affecting reproducibility of experimental results.
8. Biosafety issues: the aerosol generated in the course of grinding is likely to carry pathogens and thus pose a threat to worker health.

It is imperative to provide a novel biological sample grinding method to address the aforesaid issues.

### SUMMARY OF THE INVENTION

To achieve the above and other objectives, the disclosure provides a sample grinding kit, comprising: a sample tube for containing a sample; and a grinding element having a spiral portion enclosing the grinding element and a connect portion disposed at a top of the grinding element, the spiral portion being of a diameter less than or equal to an inner diameter of the sample tube, and the connect portion connecting the grinding element and an external power source.

Preferably, the sample tube is made of plastic, metal or ceramic.

Preferably, the grinding element is made of plastic, metal or ceramic.

Preferably, the spiral portion has a pitch of 1 to 15 mm.

Preferably, further comprise an O-ring disposed between the grinding element and the sample tube.

Preferably, the sample comprises an animal sample, plant sample,
microbiological sample, soil sample or water sample.

Another objective of the disclosure is to further provide a sample grinding device, comprising: a base; a support disposed on the base; and a rotation mechanism disposed on the support and having a connecting element for connecting to the grinding element of the sample grinding kit.

Preferably, the rotation mechanism is a gear assembly, belt drive system, chain set, electric driving mechanism or magnetic transmission mechanism.

Preferably, the connecting element corresponds in quantity to the grinding element.

Preferably, the rotation mechanism comprises 1 to 96 connecting elements.

Yet another objective of the disclosure is to further provide a sample grinding method, comprising steps of: mixing a sample and a grinding auxiliary material and then placing the mixture in a sample tube; coupling a grinding element and the sample tube together and connecting the grinding element and a rotation mechanism of the sample grinding device; and rotating, by the rotation mechanism, the grinding element to grind the sample with the grinding auxiliary material.

Preferably, the grinding auxiliary material is glass beads, steel beads, ceramic beads or a synthetic grinding auxiliary material.

Owing to the aforesaid technical features, the disclosure provides a sample grinding kit, sample grinding device and sample grinding method to achieve high-performance, precise and stable sample grinding. The disclosure is preferably suitable for biological sample grinding, and even more preferably suitable for the sample pre-processing of nucleic acid extraction to obtain loss-free, contamination-free, and evenly ground biological samples easily, preclude equipment wear and tear or contamination, control the temperature of biological samples, and ensure research safety.

The above and other objectives, features and advantages of the disclosure are illustrated by preferred embodiments, depicted by accompanying drawings, and described below.

### BRIEF DESCRIPTION OF THE DRAWINGS

Many specific details are included in the detailed description below to explain the disclosure and enable persons skilled in the art to thoroughly understand embodiments of the disclosure. However, obviously, one or more embodiments may be implemented in the absence of the specific details, otherwise conventional structures and process flows shown in the accompanying drawings are presented schematically for brevity.
FIG. 1 is a schematic view of a grinding element in a sample grinding kit according to an embodiment of the disclosure.
FIG. 2 is a schematic view of a sample tube in the sample grinding kit according to an embodiment of the disclosure.
FIG. 3 is a schematic view of a combination of the grinding element and the sample tube in the sample grinding kit according to an embodiment of the disclosure.
FIG. 4 is a block diagram of a sample grinding device according to an embodiment of the disclosure.
FIG. 5 is a schematic view of a rotation mechanism and the sample grinding kit coupled together according to an embodiment of the disclosure.
FIG. 6 is a flow chart of a sample grinding method according to an embodiment of the disclosure.
FIG. 7 shows a picture of glass beads functioning as a grinding auxiliary material according to a specific embodiment of the disclosure.

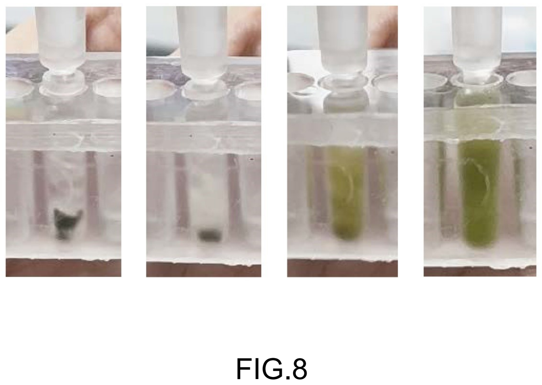

FIG. 8 shows pictures of grinding plant samples according to a specific embodiment of the disclosure.

### DETAILED DESCRIPTION OF THE EMBODIMENTS

The embodiments of the disclosure are depicted by drawings and fully described below. Although the embodiments can be implemented in various forms, this is not the only way of implementing or using the claimed invention and thus shall not be interpreted in a manner to be restrictive of the embodiments. Disclosed herein are features of multiple specific embodiments, steps of a method for constructing and operating the specific embodiments, and sequences of the steps. However, identical or equivalent functions and step sequences may also be achieved in any other specific embodiments. The embodiments enable the subject matters of the disclosure to be thoroughly and completely disclosed to sufficiently inform persons skilled in the art of the spirit of the disclosure. Similar reference numerals in the drawings denote similar components. Conventional functions or structures are not described in detail below in order not to reiterate unnecessary details of the embodiments.

Unless otherwise defined, all the technical terms and jargon used herein each carry the same meaning as usually understood by persons skilled in the art. In case of a conflict, definitions specified herein shall prevail.

Every singular noun used herein can encompass the plural form of the noun, and vice versa, provided that doing so does not render the context contradictory. The expressions "at least one" and "one or more" used herein and used in the claims have the same meaning, and both mean one, two, three or more.

The expression "consisting essentially of" used herein is intended to define a composition, method or device, including the materials, steps, features, ingredients or components other than what are expressly stated, providing a restrictive condition, i.e., the additional materials, steps, features, ingredients or components do not significantly affect basic and novel features of an invention claimed. The expression "consisting essentially of" lies between the expressions "comprising" and "consisting of" in terms of scope.

Although all parameters and numerical value ranges for defining wide scope of the disclosure are approximations, related numerical values are stated as precisely as possible in specific embodiments. However, intrinsically, every numerical value inevitably comes with some errors arising from a standard deviation associated with individual testing methods. The adverb "approximately" used hereunder usually means that the difference between an actual numerical value and a specific numerical value or range is less than 10%, 5%, 1% or 0.5%. Alternatively, the adverb "approximately" used hereunder means that an actual numerical value differs from the mean by an acceptable standard deviation or less, and the standard deviation is defined by persons skilled in the art. Apart from the embodiments, or unless otherwise specified, all numerical ranges, quantity, numerical values, and percentages (for example, descriptive of the amount of a material used, time period, temperature, operation conditions, quantitative proportion, and the like) used hereunder shall be interpreted in a manner to mean that they are modified with the adverb "approximately". Therefore, unless otherwise specified, all numerical parameters disclosed hereunder and disclosed in the claims are approximations and are subject to changes as needed. At the very least, the numerical parameters must be interpreted as numerical values characterized by significant figures and obtained by a general carry system. In this regard, a numerical value range is expressed as starting from an endpoint and ending at another endpoint or as lying between two endpoints. Unless otherwise specified, every numerical value range includes endpoints.

According to an embodiment of the disclosure, provided is a sample grinding kit, comprising: a sample tube for containing a sample; and a grinding element having a spiral portion enclosing the grinding element and a connect portion disposed at a top of the grinding element, the spiral portion being of a diameter less than or equal to an inner diameter of the sample tube, and the connect portion connecting the grinding element and an external power source. In a preferred embodiment, the sample tube is made of plastic, metal or ceramic, and the grinding element is made of plastic, metal or ceramic. The metal is resilient metal, such as stainless steel, which manifests satisfactory resilient deformation capability and corrosion-resistant properties. The ceramic is wear-resistant ceramic.

In an embodiment, the spiral portion has a pitch of 1 to 15 mm, preferably 5 mm, which is not limited to the embodiment of the disclosure and is adjustable as needed. In another embodiment, disposed between the grinding element and the sample tube is an O-ring which is made of a soft material, such as rubber and silicone, and adapted to not only hermetically seal the sample tube to prevent overflow of the generated aerosol or sample but also reduce vibration in the course of sample grinding.

According to an embodiment of the disclosure, provided is a sample grinding device, comprising: a base; a support disposed on the base; and a rotation mechanism disposed on the support and having a connecting element for connecting to the grinding element of the sample grinding kit to transmit rotation power. The rotation mechanism for generating rotation power is a gear assembly, belt drive system, chain set, electric driving mechanism or magnetic transmission mechanism or any power source capable of achieving rotation effect. In a specific embodiment, the rotation mechanism has 1, 2, 3, 4, 5, 6, 7 or 8 connecting elements. In another embodiment, the rotation mechanism has 16, 24, 48, 96 connecting elements; however, the disclosure is not limited thereto. According to an embodiment of the disclosure, the sample grinding device drives one single rotation mechanism performing grinding independently or drives multiple rotation mechanisms grinding multiple samples simultaneously. Depending on the samples to grind, the grinding process achieves optimization of grinding effect through fixed-speed rotation, variable-speed rotation or intermittent rotation. In another specific embodiment, the rotation mechanism is Maelstrom Switch 8.

According to an embodiment of the disclosure, provided is a sample grinding method, comprising steps of: mixing a sample and a grinding auxiliary material and then placing the mixture in a sample tube; coupling a grinding element and the sample tube together and connecting the grinding element and the rotation mechanism of the sample grinding device through a connecting element; and rotating, by the rotation mechanism, the grinding element to allow the grinding element to grind the sample with the grinding auxiliary material. The grinding auxiliary material is glass beads, steel beads, ceramic beads or a synthetic grinding auxiliary material. The synthetic grinding auxiliary material is a polymeric or composite material, for example, polyethylene terephthalate (PET) or glass fiber reinforced plastic (GFRP). The ratio of the sample to the grinding auxiliary material is 1:10~10:1, preferably 6:1.

The subject matters of the disclosure are illustrated by embodiments, depicted by accompanying drawings, and described below.

### Sample grinding kit in embodiment 1 of the disclosure

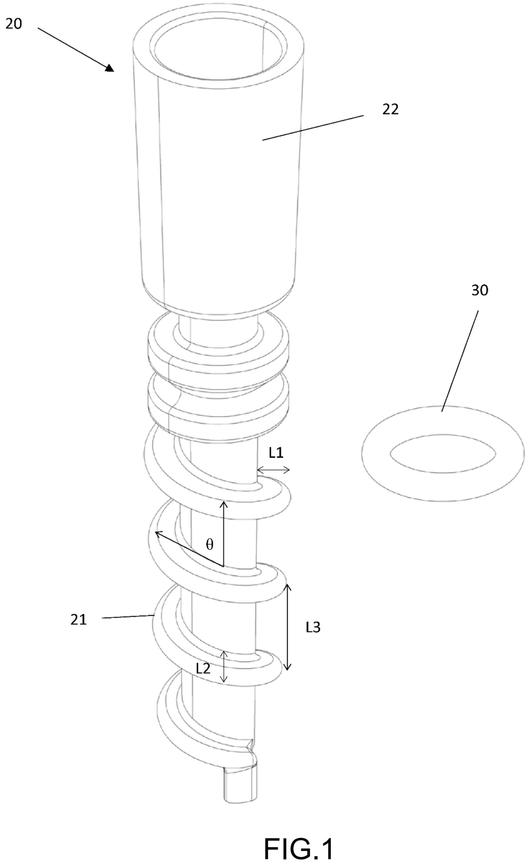

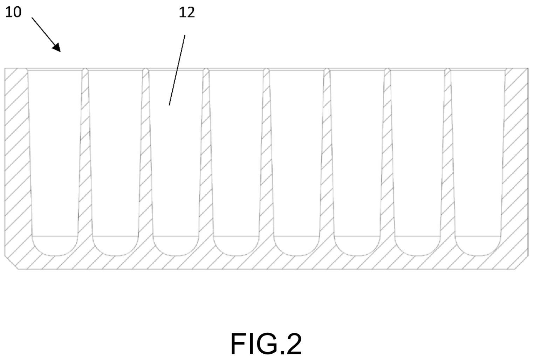

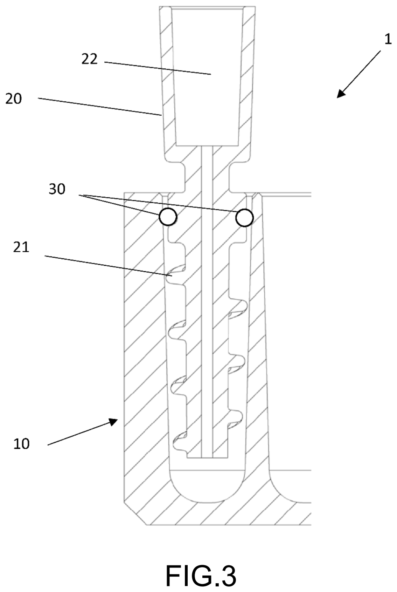

Refer to FIG.1 to FIG.3. FIG. 1 is a schematic view of a grinding element 20 in a sample grinding kit 1 according to an embodiment of the disclosure. FIG. 2 is a schematic view of a sample tube 10 in the sample grinding kit 1 according to an embodiment of the disclosure. FIG. 3 is a schematic view of a combination of the grinding element 20 and the sample tube 10 in the sample grinding kit 1 according to an embodiment of the disclosure.

According to the disclosure, the sample grinding kit 1 comprises the grinding element 20 and the sample tube 10. Referring to FIG. 1, the grinding element 20 has an upper end provided with a connect portion 22 and a lower end provided with a spiral portion 21. The spiral portion 21 has a width L1 of 1.5 mm, a thickness L2 of 1 mm, a pitch L3 of 5 mm, and a tilt angle θ of 30°. The sample tube 10 comprises eight sample slots 12. The sample tube 10 has a diameter of 10 mm and a height of 40 mm. The grinding element 20 is made of plastic. The sample tube 10 is made of plastic. The number of the sample slots 12 of the sample tube 10 is not limited to the aforesaid embodiment of the disclosure and thus can be adjusted by users as needed. An O-ring 30 (shown in FIG. 1) is disposed at the upper end of the spiral portion 21 of the grinding element 20 and made of rubber to isolate the sample from the surroundings, lower contamination risks, and reduce vibration during sample grinding.

### Sample grinding device in embodiment 2 of the disclosure

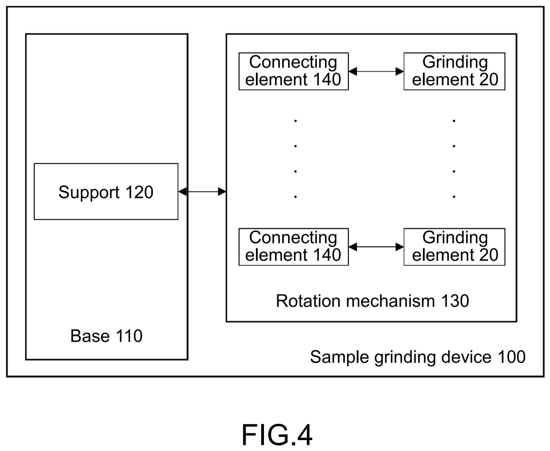

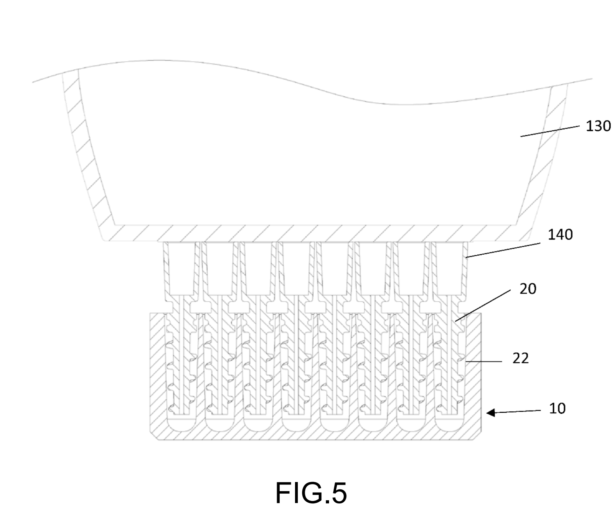

Refer to FIG.4 and FIG.5. FIG. 4 is a block diagram of a sample grinding device 100 according to an embodiment of the disclosure. FIG. 5 is a schematic view of a rotation mechanism 130 and the sample grinding kit 1 coupled together according to an embodiment of the disclosure.

Referring to FIG. 4, the sample grinding device 100 comprises: a base 110; a support 120 disposed on the base 110; and a rotation mechanism 130 disposed on the support 120 and having a connecting element 140 for connecting to the grinding element 20 of the sample grinding kit 1. The rotation mechanism 130 is Maelstrom Switch 8 and has eight connecting elements 140. In this embodiment, owing to the rotation mechanism 130, when a mechanism for providing rotation power is a gear assembly, every two adjacent ones of the connecting elements 140 rotate in opposite directions and thus have to coordinate with the grinding element 20 of the spiral portion 21 in different directions; however, the disclosure is not limited thereto, as the connecting elements 140 of the rotation mechanism 130 may also rotate in the same direction. For example, when a mechanism for providing rotation power is a belt drive system or a chain set, the connecting elements 140 rotate in the same direction. If a mechanism for providing rotation power is an electric driving mechanism or a magnetic transmission mechanism, the directions in which the connecting elements 140 rotate can be adjusted as needed and thus are not restricted to the aforesaid embodiment.

### Sample grinding method in embodiment 3 of the disclosure

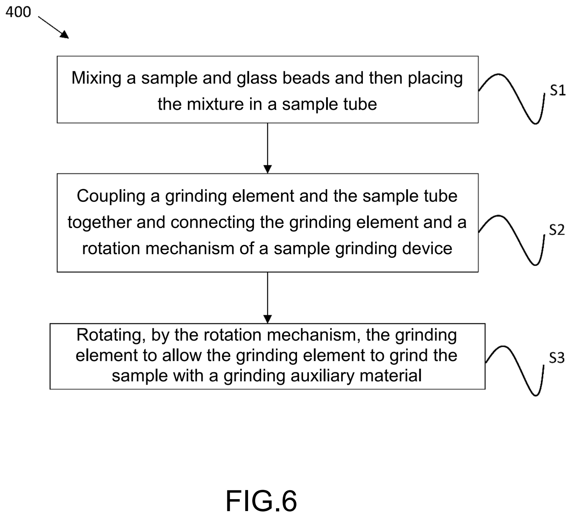

FIG. 6 is a flow chart of a sample grinding method 400 according to an embodiment of the disclosure.

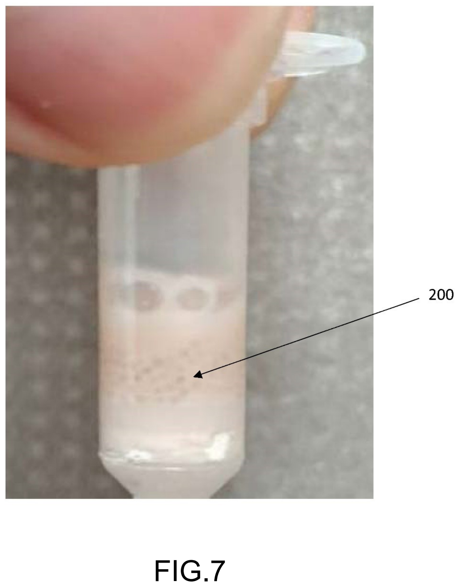

The sample grinding method 400 comprises the steps as follows: S1 mixing a sample and a grinding auxiliary material 200 and then placing the mixture in a sample tube 10; S2 coupling a grinding element 20 and the sample tube 10 together and connecting the grinding element 20 and a rotation mechanism 130 of the sample grinding device 100 through a connecting element 140; and S3 rotating, by the rotation mechanism 130, the grinding element 20 to allow the grinding element 20 to grind the sample with the grinding auxiliary material 200. Preferably, the grinding auxiliary material 200 comprises glass beads or steel beads and is 0.3 to 0.7 mm in size. The grinding auxiliary material 200 and the sample are mixed in a manner such that the ratio of the weight of the grinding auxiliary material 200 to the weight of the sample in the mixture is 6:1. Referring to FIG. 7, in this aspect, the grinding auxiliary material 200 comprises glass beads which are 0.7 mm in size, and the ratio of the weight of the grinding auxiliary material 200 to the weight of the sample in the mixture is 6:1.

Embodiments 3: Integrating the sample grinding method of the disclosure into the application of nucleic acid extraction.

In this embodiment, the grinding method of the disclosure is compared with a conventional grinding technique.

In this embodiment, the grinding method of the disclosure entails adding 0.3 g of the grinding auxiliary material 200 (glass beads), 200 mM DTT (Dithiothreitol) and 1 ml of a buffer solution to a sample (palm leaf) of a weight of 50 mg, then rotating the mixture at 500 rpm for 2 minutes by the rotation mechanism 130 (for example, Maelstrom Switch 8) through the grinding element 20 and the grinding auxiliary material 200 to grind the sample. After being ground, the sample reaches a temperature of 40°C approximately. Next, 10 µl of Proteinase K (PK) is added to the sample to undergo incubation at 65°C for 30 minutes to achieve protein degradation, allowing 500 µl of supernatant to be obtained. The total operation duration includes standstill duration and is equal to 1 hour approximately. Upon completion of extraction, the nucleic acid thus obtained has a concentration of 138.3 ng/µl, with A260/A280 being 1.93, and A260/A230 being 1.68.

The conventional grinding technique entails adding 0.3 g of the grinding auxiliary material 200 (glass beads), 200 mM DTT, 1 ml of a buffer solution and liquid nitrogen to a sample (palm leaf) of a weight of 50 mg to perform manual grinding. Upon completion of the grinding process, the sample reaches a temperature of -196°C. Then, 10 µl of Proteinase K (PK) is added to the sample to undergo incubation at 65°C for 30 minutes to achieve protein degradation, allowing 500 µl of supernatant. The total operation duration is approximately 1 hour, exclusive of extraction duration on the centrifuge. Upon completion of extraction, the nucleic acid thus obtained has a concentration of 83 ng/µl, with A260/A280 being 1.87, and A260/A230 being 1.27.

The findings of the comparison are presented in Table 1 below.

**Table 1**

| | **Present invention** | **Liquid nitrogen grinding** |
|---|---|---|
| **Sample** | Palm leaf, 50 mg | Palm leaf, 50 mg |
| **Grinding device** | Grinding system of the present disclosure, 500 rpm, 2 minutes | Manual grinding, 5 minutes |
| **Glass bead amount** | Buffer solution: 1 ml, | Buffer solution: 1 ml, |
| | glass beads: 0.3 g, | glass beads: 0.3 g, |
| | 200 mM DTT | 200 mM DTT |
| **Temperature after grinding** | Approximately 40°C | Approximately -196°C |
| **Incubation** | Proteinase K: 10 ul | Proteinase K: 10 ul |
| | 30 mins @ 65°C | 30 mins @ 65°C |
| **Product collection** | 500 ul of supernatant | 500 ul of supernatant |
| **Outcome** | Conc.: 138.3 ng/ul, | Conc.: 83 ng/ul, |
| | 260/280: 1.93 | 260/280: 1.87 |
| | 260/230: 1.68 | 260/230: 1.27 |
| **total operation duration** | approximately 1 hour (exclusive of extraction duration on the centrifuge) | approximately 1 hour (exclusive of extraction duration on the centrifuge) |

Therefore, the sample grinding kit, sample grinding device and sample grinding method provided by the disclosure are effective in grinding biological samples thoroughly, increasing nucleic acid extraction rate, and preventing sample contamination with an O-ring. The conventional grinding technique requires liquid nitrogen to break open the cell walls and membranes of biological samples. The sample grinding kit, sample grinding device and sample grinding method of the disclosure dispense with hazardous substances, such as liquid nitrogen, and thus enhance user operation safety. Since the sample grinding kit of the disclosure can adjust rotation speed during a sample grinding process to achieve temperature control, the sample grinding process itself has a heating effect, dispensing with the need to perform an additional heating process subsequently.

The aforesaid embodiments are illustrative rather than restrictive of the disclosure. All equivalent amendments and changes made to the embodiments without departing from the spirit and scope of the disclosure shall be deemed falling within the scope of the claims of the disclosure.

## Claims

1. A sample grinding kit (1), comprising:
a sample tube (10) for containing a sample; and
a grinding element (20) having a spiral portion (21) enclosing the grinding element (20) and a connect portion (22) disposed at a top of the grinding element (20), the spiral portion (21) being of a diameter less than or equal to an inner diameter of the sample tube (10), and the connect portion (22) connecting the grinding element (20) and an external power source.

2. The sample grinding kit (1) of claim 1, wherein the sample tube (10) is made of plastic, metal or ceramic.

3. The sample grinding kit (1) of claim 1, wherein the grinding element (20) is made of plastic, metal or ceramic.

4. The sample grinding kit (1) of claim 1, wherein the spiral portion (21) has a pitch of 1 to 15 mm.

5. The sample grinding kit (1) of claim 1, further comprise an O-ring (30) disposed between the grinding element (20) and the sample tube (10).

6. The sample grinding kit (1) of claim 1, wherein the sample comprises an animal sample, plant sample, microbiological sample, soil sample or water sample.

7. A sample grinding device (100), comprising:
a base (110);
a support disposed on the base (110); and
a rotation mechanism (130) disposed on the support and having a connecting element (140) for connecting to the grinding element (20) of the sample grinding kit (1) of any one of claims 1-6 to transmit rotation power.

8. The sample grinding device (100) of claim 7, wherein the rotation mechanism (130) is a gear assembly, belt drive system, chain set, electric driving mechanism or magnetic transmission mechanism.

9. The sample grinding device (100) of claim 7, wherein the connecting element (140) corresponds in quantity to the grinding element (20).

10. The sample grinding device (100) of claim 9, wherein the rotation mechanism (130) comprises 1 to 96 connecting elements (140).

11. A sample grinding method (400), comprising steps of:
mixing a sample and a grinding auxiliary material (200) and then placing the mixture in the sample tube (10) of any one of claims 1-6;
coupling the grinding element (20) and the sample tube (10) together and connecting the grinding element (20) and the rotation mechanism (130) of the sample grinding device (100) of any one of claims 7-10 through the connecting element (140); and
rotating, by the rotation mechanism (130), the grinding element (20) to allow the grinding element (20) to grind the sample with the grinding auxiliary material (200).

12. The method (400) of claim 11, wherein the grinding auxiliary material (200) is glass beads, steel beads, ceramic beads, or a synthetic grinding auxiliary material (200).
